Europäisches Patentamt

European Patent Office

Office européen des brevets

(11) Publication number: **0 172 612**
**A1**

# (12) EUROPEAN PATENT APPLICATION

(21) Application number: **85304004.6**

(22) Date of filing: **05.06.85**

(51) Int. Cl.⁴: **C 07 C 149/42**
**C 07 C 148/00**

(30) Priority: **25.06.84 US 624532**

(43) Date of publication of application:
**26.02.86 Bulletin 86/9**

(84) Designated Contracting States:
**AT BE CH DE FR GB IT LI LU NL SE**

(71) Applicant: **ETHYL CORPORATION**
**Ethyl Tower 451 Florida Boulevard**
**Baton Rouge Louisiana 70801(US)**

(72) Inventor: **Ranken, Paul Frederick**
**10345 Westwood Avenue**
**Baton Rouge Louisiana 70809(US)**

(74) Representative: **Bizley, Richard Edward et al,**
**BOULT, WADE & TENNANT 27 Furnival Street**
**London EC4A 1PQ(GB)**

(54) Preparation of (hydrocarbylthio)aromatic amines.

(57) (Hydrocarbylthio)aromatic amines, such as (alkyl-thio)anilines, are prepared by heating one or more other (hydrocarbylthio)aromatic amines in the presence of a catalyst, such as aluminium chloride, to redistribute the hydrocarbylthio groups.

## PREPARATION OF (HYDROCARBYLTHIO)AROMATIC AMINES

The invention relates to (hydrocarbylthio)aromatic amines and more particularly to a process for preparing them by the redistribution of other (hydrocarbylthio)aromatic amines.

It is known that various (hydrocarbylthio)aromatic amines are useful as synthetic intermediates, for example in the preparation of biologically-active materials or polyurethanes, and the present invention sets out to provide a novel process for preparing (hydrocarbylthio)aromatic amines.

It is known that such compounds can be prepared by reacting appropriate aromatic amines with hydrocarbyl disulfides in the presence of suitable catalysts, such as aluminum chloride. Unfortunately, these syntheses typically result in the formation of mixtures of (hydrocarbylthio)aromatic amines and it would also be desirable to be able to treat these products to increase the yield of any particular (hydrocarbylthio)aromatic amine.

This invention provides a novel process for preparing (hydrocarbylthio)aromatic amines by the redistribution of other (hydrocarbylthio)aromatic amines caused by heating one or more (hydrocarbylthio)aromatic amines in the presence of a catalyst.

(Hydrocarbylthio)aromatic amines used as starting materials in the practice of the invention are aromatic amines having one or more hydrocarbylthio groups attached to an aromatic ring which optionally bears one or more inert substituents, i.e., substituents inert to the reaction conditions, in the positions unsubstituted by amino or hydrocarbylthio groups.

The hydrocarbylthio groups of the (hydrocarbylthio)aromatic amines may be aliphatic, cycloaliphatic, or aromatic, for example methylthio, ethylthio, butylthio, cyclopentylthio, cyclohexylthio, benzylthio, p-tolylthio or p-chlorophenylthio; but they are preferably alkylthio groups, most preferably alkylthio groups containing 1-6 carbons.

The amine substrate of the (hydrocarbylthio)aromatic amine may be a mono- or polynuclear aromatic amine, optionally bearing inert substituents, i.e., substituents inert to the reaction conditions. In general, these aromatic amines are (1) compounds having at least one amino group attached to a carbocyclic or heterocyclic ring of an aromatic compound containing one or more simple and/or fused rings, for example benzene, naphthalene, anthracene, pyrrole, pyridine or indole rings or (2) reactive heterocyclic amines, for example pyrrole, indole or imidazole, optionally bearing substituents inert to the reaction conditions, for example one or more additional amino groups or substituents such as, e.g., chloro, fluoro, alkyl, aryl, alkaryl, or aralkyl groups on any positions other than those to be substituted by hydrocarbylthio groups. In the case of coupled aromatic rings, the rings may be directly attached to one another or may be coupled through a bridge such as an oxygen, sulfur, sulfoxide, sulfone, alkyl, or other hydrocarbon link for example. Examples of suitable aromatic amine substrates are 4,4'-methylenedianiline, 4-(phenylthio)aniline, 1,3-dimethylpyrrole, 1-methylpyrrole, 2-aminobiphenyl, 4-phenoxyaniline, 7-methylindole, aminobenzenes containing one or two amino groups, such as aniline, 4-butylaniline, 4-methylaniline, 4-chloroaniline, 2-ethylaniline, N-methylaniline, 2,4- and 2,6-di-aminotoluenes or 2,6-diamino-1-ethylbenzene.

Catalysts that can be used in the practice of the invention are generally Lewis acid catalysts, for example metal halides, e.g., aluminum chloride, boron trifluoride, ferric chloride or zinc chloride; metal alkyls, e.g., triethylaluminum, diethylaluminum chloride or ethyl aluminum dichloride; or the organometallic compounds derived from the reaction of an aromatic amine with the metal halides, metal alkyls, and reactive metals such as aluminum for example. The preferred catalysts are the metal halides, such as aluminum chloride, boron trifluoride, and boron trichloride, with aluminum chloride being especially preferred.

The process of the invention is conducted by contacting the (hydrocarbylthio)aromatic amine with a catalytic amount, e.g., 0.01-0.5, preferably 0.01-0.2, mol of the catalyst per mole of (hydrocarbylthio)aromatic amine, and heating the reaction mixture at a suitable temperature, e.g., 100-300°C., until the hydrocarbylthio groups have been redistributed and the desired (hydrocarbylthio)aromatic amine has been formed. If desired, the reaction can be conducted in the presence of an added aromatic amine, which can be useful in reacting with hydrocarbylthio groups of a poly(hydrocarbylthio)aromatic amine when a (hydrocarbylthio)aromatic amine containing fewer hydrocarbylthio groups is desired.

After completion of the redistribution reaction, the desired (hydrocarbylthio)aromatic amine can be isolated by fractionation. Alternatively, the reaction mixture can be reacted with a suitable hydrocarbyl disulfide to provide additional quantities of the desired (hydrocarbylthio)aromatic amine.

EXAMPLE

A mixture of 13.92 g (0.1 mol) of 2-(methylthio)aniline and 0.893 g (0.0067 mol) of aluminum chloride was stirred at 165-170°C. in a nitrogen atmosphere for 18 hours. The reaction mixture was cooled, diluted with 50 ml of ether, hydrolyzed with 10 ml of 1N sodium hydroxide, and then extracted with 10 ml of saturated aqueous sodium chloride. The solvent was removed under reduced pressure (40 mm) and the residue distilled (0.35 mm) to give 9.4 g of distillate having a boiling point of 30-115°C. Analysis by gas chromatography and mass spectroscopy showed:

21 area % aniline
58 area % 2-(methylthio)aniline
7 area % 4-(methylthio)aniline
7 area % 2,4-di(methylthio)aniline
6 area % 2,6-di(methylthio)aniline

Included within the present invention are processes for preparing a biologically active material, polyurethane or other industrial product in which the process of the invention is used to prepare a (hydrocarbylthio)aromatic amine intermediate, and the use of the process of the invention in the preparation of a biologically active material, polyurethane or other industrial product.

Also provided is the use of the process of the invention as an intermediate synthetic process. By "intermediate synthetic process" is meant a process used as an intermediate stage in a synthetic process.

CLAIMS:

1. A process which comprises heating one or more (hydrocarbylthio)aromatic amines in the presence of a catalyst to redistribute the hydrocarbylthio groups.

2. A process as claimed in claim 1 wherein the amine moiety of the (hydrocarbylthio)aromatic amine is an aminobenzene or a heterocyclic amine.

3. A process as claimed in claim 2 wherein the aminobenzene is aniline.

4. A process as claimed in claim 1 wherein the (hydrocarbylthio)aromatic amine is an (alkylthio)aromatic amine.

5. A process as claimed in claim 4 wherein the alkylthio)aromatic amine is an (alkylthio)aniline having 1-6 carbons in the alkyl group.

6. A process as claimed in any one of the preceding claims wherein the catalyst is a metal halide, metal alkyl, or an organometallic compound derived by reacting an aromatic amine with a metal halide, metal alkyl, or reactive metal.

7. A process as claimed in claim 6 wherein the catalyst is aluminum chloride.

8. A process as claimed in any one of the preceding claims wherein the reaction is conducted at a temperature of 100-300$^{\circ}$C.

9. A process as claimed in claim 1 wherein an (alkylthio)aniline is heated at 100-300$^{\circ}$C. in the presence of a catalytic amount of aluminum chloride.

10. A process for preparing a biologically active material, polyurethane or other industrial product in which a process as claimed in any one of the preceding claims is used to prepare a (hydrocarbylthio)aromatic amine intermediate; the use of a process as claimed in any one of the preceding claims in the preparation of a biologically active material, polyurethane or other industrial product; or the use of a process as claimed in any one of the preceding claims as an intermediate synthetic process.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 85, no. 23, 6th December 1976, page 467, column 1, abstract no. 176786e, Columbus, Ohio, US; T. KAWATA et al.: " Catalytic rearrangement of O,S-dialkyl dithiocarbonates to S,S-dialkyl dithiocarbonates. II. The effects of structure of O,S-dialkyl dithiocarbonates, catalysts and solvents", & YAKUGAKU ZASSHI 1976, 96(7), 832-840 <br><br>--- | 1 | C 07 C 149/42 <br> C 07 C 148/00 |
| A | CHEMICAL ABSTRACTS, vol. 68, no. 17, 22nd April 1968, page 7467, column 1, abstract no. 77491x, Columbus, Ohio, US; J. NYITRAI et al.: "S-alkyl vs. S-heterocyclic bond cleavage during desulfuration under rearrangement of S-alkyl-5,5-diphenyl-4-thiohydantoins by aluminium chloride", & ACTA CHIM. ACAD.SCI. HUNG. 1967, 54(2), 209-211 <br><br>----- | 1 | |

**TECHNICAL FIELDS SEARCHED (Int. Cl.4)**

C 07 C 149/42
C 07 C 148/00

The present search report has been drawn up for all claims

| Place of search | Date of completion of the search | Examiner |
|---|---|---|
| BERLIN | 26-09-1985 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82